(19) Europäisches Patentamt
European Patent Office
Office européen des brevets

(11) Numéro de publication : **0 437 388 B1**

(12) **FASCICULE DE BREVET EUROPEEN**

(45) Date de publication du fascicule du brevet :
**30.03.94 Bulletin 94/13**

(21) Numéro de dépôt : **91400020.3**

(22) Date de dépôt : **08.01.91**

(51) Int. Cl.$^5$ : **A23K 1/16,** A23K 1/18,
A61K 9/16, A61K 9/20,
A61K 9/52

(54) **Procédé d'incorporation dans des pellets de principes actifs protégés contre la dégradation dans la panse des ruminants.**

(30) Priorité : **09.01.90 FR 8917305**

(43) Date de publication de la demande :
**17.07.91 Bulletin 91/29**

(45) Mention de la délivrance du brevet :
**30.03.94 Bulletin 94/13**

(84) Etats contractants désignés :
**AT BE CH DE DK ES FR GB GR IT LI LU NL SE**

(56) Documents cités :
**EP-A- 0 100 974
US-A- 3 413 118
US-A- 4 066 754**

(73) Titulaire : **RHONE-POULENC NUTRITION ANIMALE
Rue Marcel Lingot
F-03600 Commentry (FR)**

(72) Inventeur : **Annonier, Claude
18 Allée des Saules
F-03410 Premilhat (FR)**
Inventeur : **Autant, Pierre
14 rue de Pourcheroux
F-03600 Commentry (FR)**
Inventeur : **Porte, Hugues
6 Chemin de Crépieux
F-69300 Caluire (FR)**
Inventeur : **Ruel, Jacques
6 rue du Général Leclerc
F-95210 Saint-Gratien (FR)**

(74) Mandataire : **Le Pennec, Magali et al
RHONE-POULENC RORER SA, Direction des
Brevets, 20 Avenue Raymond Aron
F-92165 Antony Cédex (FR)**

## Description

La présente invention concerne un procédé d'incorporation dans des pellets de principes actifs. Elle concerne plus particulièrement l'incorporation dans des pellets de principes actifs protégés vis à vis des enzymes de la panse des ruminants. Elle concerne encore plus particulièrement la préparation de pellets destinés à être incorporés à l'alimentation des ruminants.

On entend par "pellet" un granulé nutritif pour les animaux obtenu soit par extrusion à travers une filière d'un mélange d'aliments soit par une autre technique. Ils se présentent sous forme de bâtonnets de préférence cylindrique présentant notamment des dimensions moyennes de 4 à 20 mm de long et de 4 à 10 mm de diamètre. Ils sont largement utilisés, car ils sont faciles à manipuler, à administrer et non poussiérants.

Le problème que cherche à résoudre la présente invention consiste à incorporer dans des "pellets" destinés à l'alimentation animale des granulés contenant des composés utiles à leur alimentation et/ou à la thérapeutique de ces animaux, mais qui ne peuvent subir la pelletisation du fait de leur sensibilité à la température, à la pression et au cisaillement. En effet, les appareils de pelletisation sont des appareils qui permettent la mise en forme par forçage d'un mélange alimentaire à travers une filière en présence de vapeur d'eau.

Ce mélange est passé à travers une plaque perforée (la filière) par le moyen d'une presse qui force la masse à extruder à travers les orifices de la plaque. A la sortie de la filière, les cylindres obtenus sont coupés par un moyen mécanique ou spontanément. Lors du passage forcé à travers la filière, les produits à faible résistance thermique ou mécanique subissent une dégradation due à la fois à la pression exercée et à la chaleur provoquée à la fois par le frottement et par l'addition de vapeur favorable à la cohésion des divers ingrédients farineux des aliments de base.

Ce problème a déjà été évoqué dans diverses antériorités. On peut citer parmi ces antériorités la demande de brevet européen publiée sous la numéro EP 231 817 qui décrit un procédé de préparation "d'aliments agglomérés" à base de vitamines ou de composés indispensables à la santé humaine ou animale. Dans l'art antérieur, il était connu que lorsque ces composés subissent une élévation de température lors du passage dans des appareils de pelletisation, ils sont détruits au moins partiellement. En effet, les composés à faible résistance thermique ou mécanique sont souvent mélangés pour leur mise en forme à de la mélasse et subissent l'action de la vapeur afin d'assurer leur cohésion. L'action simultanée de la vapeur et de la pression les détruit. La demande de brevet EP 231 817 a permis de résoudre le problème de fabrication de ces "aliments agglomérés" par pulvérisation sur un noyau de base d'une solution ou d'une suspension de la vitamine ou des vitamines ou du médicament et éventuellement d'un corps gras. Ce procédé ne permet qu'un enrobage du corps de base par la vitamine avec ou sans corps gras. Ledit procédé est facile à mettre en oeuvre à partir de produits pulvérulents ou liquides tels que les vitamines ou les médicaments. Ces produits sont ainsi faciles à mettre en oeuvre sous forme de solution ou d'une suspension. Il n'en est pas de même lorsque l'on forme des "granulés nutritifs agglomérés" dans lesquels la matière sensible à la chaleur et/ou à la compression se présente elle-même sous forme de granulés de diamètre moyen d'environ 0,3 à 5 mm ce qui est le cas dans la présente invention.

Il est également décrit dans le brevet français publié sous le numéro FR 2 338 653 un procédé de préparation de produits alimentaires sur lesquels sont pulvérisés une suspension d'enzyme de préférence de protéases dans une matière grasse liquide ou fondue. Ce procédé apporte le même enseignement que la demande européenne préalablement évoquée et ne permet d'obtenir que des "pellets" sur lesquels la substance thermosensible est déposée en périphérie par un phénomène de pulvérisation.

Il est connu selon le brevet EP-A-100 974 de préparer des pellets de granulés stables à la chaleur et à la vapeur d'eau contenant un complexe de zinc et d'amino acide stable dans le rumen. La stabilité au rumen et à la chaleur est apportée par la modification de la structure chimique de l'amino acide.

Il est encore connu selon le brevet US 4 066 754 de préparer des bolus contenant des granulés d'un principe actif et d'un matériau dense protégé par un enrobage de zéine, de la zéine pulvérulante et un agent lubrifiant. Ces granulés ne contiennent pas de matière fusible et la protection n'est pas détruite par la compression.

Il est enfin connu selon le brevet US 3 413 118 de préparer des granulés d'urée ou de sels d'ammonium libérables dans le rumen qui sont enrobés d'une émulsion et de luzerne et peuvent être pelletisés. Ces granulés ne répondent pas aux objectifs de la présente invention car ils ne sont pas rumen-résistant.

Le problème qu'a cherché à résoudre la présente invention est la mise sous forme de "pellets", directement assimilables par les animaux, d'additifs destructibles au niveau de la panse des ruminants tels que les aminoacides, les vitamines ainsi que d'autres additifs nutritionnels ou médicamenteux.

Il est connu par exemple, que certains aminoacides sont essentiels dans l'alimentation des ruminants car ils sont limitants dans l'apport nutritionnel journalier. Il en est ainsi notamment de la méthionine et de la lysine. Ces substances, lorsqu'elles sont administrées aux ruminants par voie orale sont détruites dans la panse par

2

l'action des enzymes digestives et des microorganismes présents dans cet organe. Il a été découvert que pour que ces composés soient utilisables et bénéfiques pour l'animal, il fallait les protéger par une substance leur permettant de traverser le rumen sans dommage mais délitable au niveau ou après la caillette de façon à libérer la substance active dans l'intestin et permettre ainsi son passage dans l'organisme.

Généralement, les compositions d'enrobage connues sont constituées de l'association d'une substance sensible aux variations du PH, choisie en particulier parmi des copolymères basiques synthétiques, avec des substances hydrophobes qui peuvent être choisies, par exemple, parmi les acides gras ou leurs dérivés et les polymères hydrophobes. De telles compositions sont décrites, par exemple, dans les brevets français FR 78 23966 (2 401 620), FR 78 23968 (2 401 621) ou FR 81 18954 (2 514 261).

Parmi les principaux groupes d'agents d'enrobage, on peut citer à titre d'exemples les copolymères de la vinyl pyridine et du styrène avec une substance hydrophobe de préférence l'acide stéarique et/ou un polymère non hydrosoluble, par exemple l'éthylcellulose.

On peut aussi citer le groupe d'agents d'enrobage à digestion enzymatique, tels que le chitosan et/ou la zéïne, associé à une substance hydrophobe de préférence l'acide stéarique et éventuellement à un polymère non hydrosoluble de préférence l'éthylcellulose.

Les granulés de principes actifs protégés obtenus se présentent sous forme de particules plus ou moins sphériques ayant un diamètre moyen compris entre 0,3 et 5 mm, de préférence d'environ 2 mm.

Il est inenvisageable de pouvoir pulvériser des granulés de cette dimension sur des "pellets" de matière nutritive. Ces granulés ne peuvent pas non plus être introduits dans des appareils de pelletisation car l'enrobant subit des aggressions telles que l'abrasion, le cisaillement, l'élévation de température, l'adjonction de vapeur d'eau et la pression qui entraînent une dégradation au moins partielle de l'enrobant et donc une non-stabilité des principes actifs dans le rumen lors de l'ingestion par l'animal.

Il semblait donc impossible de faire ingérer aux ruminants lesdits principes actifs protégés autrement qu'en les dispersant dans l'aliment ce qui pose des problèmes d'homogénéité, de concentration et de distribution.

La présente invention a permis d'atteindre cet objectif. Elle consiste à préparer des "pellets spéciaux" contenant des principes actifs protégés contre la dégradation dans le rumen mais dégradables par l'action conjointe de la chaleur et de la pression, utilisés pour la supplémentation alimentaire et/ou médicamenteuse des ruminants caractérisés en ce qu'ils sont obtenus par mélange du principe actif protégé sous forme de granulé d'un liant choisi parmi les liants solubilisables, reticulables, les liant fusibles et éventuellement d'un agent délitant et/ou d'une charge.

Les principes actifs protégés contre l'action du rumen utilisables sous cette forme sont choisis parmi :
- les aminoacides essentiels,leurs sels, leurs dérivés et leurs analogues tels que:
    la méthionine
        la lysine.
- les vitamines
- les principes médicamenteux tels que les antibiotiques.

Les agents liants utilisables dans le cadre de la présente invention sont choisis de préférence parmi les matières alimentaires permettant une transformation liquide/solide de telle sorte qu'une forme définie proche de celle du "pellet" alimentaire puisse lui être conférée et figée.

Deux classes d'agents liants alimentaires peuvent être utilisés :
- les agents liants utilisés en milieu solvant ou dispersant,
- les agents fusibles.

Parmi les agents liants utilisés en mileu solvant ou dispersant, on peut citer :
- la classe des hydrocolloïdes tels que notamment les dérivés hydrosolubles de la cellulose tels que : la carboxyméthylcellulose, l'hydroxypropylcellulose, l'hydroxyéthylcellulose, l'hydroxyméthylcellulose.
- la classe des polysaccharides naturels ou synthétiques tels que : la gomme arabique, la gomme adragante, les carraghénates, les dextrines, l'amidon, la gomme xanthane, les alginates.
- les sucres,
- les mélasses et les vinasses,
- les lignosulfonates,
- les farines de grains ou d'algues,
- la classe des composés minéraux cristallisables tels que la chaux, le plâtre, le silicate de sodium, le carbonate de calcium et la silice.
- les gélatines,
- les protéines tannées,
- les sels de cations polyvalents des polyacides naturels ou synthétiques,
- les huiles siccatives et les mastics obtenus par association d'une huile siccative et d'une charge.

Certains liants sont utilisés avec des agents de réticulation choisis par exemple parmi: les aldéhydes pour les protéines, les sels ou oxydes de métaux di ou trivalents pour les alginates, la gomme xanthane, les mélasses,les vinasses et les autres agents de durcissement adaptés aux liants utilisés et bien connus de l'homme de l'art.

Parmi les liants fusibles, on peut citer à titre d'exemples :
- les acides et alcools gras,
- les graisses végétales et animales hydrogénées,
- les esters du glycérol,
- les paraffines,
- les cires naturelles ou synthétiques,
- les polymères synthétiques tels que les polyéthylène glycols, l'acétate de polyvinyle...

On préfère, parmi l'ensemble des liants, utiliser les mélasses, les vinasses, les acides gras, les graisses végétales ou animales hydrogénées, le plâtre et les cires paraffiniques.

On peut introduire dans le pellet spécial selon l'invention, des additifs qui permettent d'apporter au pellet les performances de densité, de résistance mécanique et de délitement rapide dans le rumen.

A titre d'additifs utiles pour la préparation des pellets spéciaux selon l'invention, on peut citer :
- les additifs minéraux tels que : la silice, les silicates, le talc, les argiles, les carbonates de calcium, les phosphates.
- les additifs issus de produits naturels tels que : les farines de grains, les résidus des industries des céréales et du bois, les tourteaux broyés, les résidus de brasserie et de fermentation diverses, des fibres végétales cellulosiques, des polysaccharides, des sucres.

Le liant complété éventuellement des additifs représente de préférence 40 à 95 % de la masse du "pellet spécial selon l'invention". La teneur en additifs minéraux ou naturels représente de 0 à 80 % en poids par rapport au liant.

Le mélange du liant et du produit protégé peut se faire en même temps que la mise en forme ou précedemment. La mise en forme peut se faire par l'intermédiaire d'une filière ou d'un moule.

La nature du liant, la température de coulée, la quantité d'additifs seront adaptées par l'homme de l'art en fonction de la qualité du pellet spécial selon l'invention désiré :
- son appétence,
- sa densité,
- sa forme,
- sa dimension,
- sa résistance mécanique,
- sa solubilité,
- son aptitude à se déliter dans le rumen.

Ils se présentent sous forme de bâtonnets de préférence cylindrique présentant notamment des dimensions moyennes de 4 à 20 mm de long et de 4 à 10 mm de diamètre.

Les pellets spéciaux selon l'invention présentent au moins trois caractéristiques qui les rendent utiles pour l'alimentation des ruminants:

1/ Ils sont miscibles en toutes proportions avec les aliments granulés généralement distribués à l'animal en supplémentation de la ration fourragère de base. C'est à dire qu'ils peuvent supporter sans démélange les différentes étapes de manutention auxquelles sont soumis les aliments granulés, et s'intègrent dans la chaine de distribution de l'aliment.

2/ La perte de protection des principes actifs protégés y est limitée lors de la mise en forme.

3/ Les "pellets spéciaux selon l'invention" ainsi réalisés se délitent rapidement dans le rumen, et évitent une perte de protection des principes actifs inclus, par mastication lors de la rumination. Les principes actifs protégés peuvent alors continuer librement le transit.

L'aptitude à la miscibilité avec les aliments granulés traditionnels dépend essentiellement de la forme et de la densité des produits réalisés et ne présente pas de difficulté pour l'homme de l'art.

Le taux de protection qui caractérise les principes protégés est égal à 100 moins le taux de libération constaté in vitro par mesure de la libération des principes actifs, après 24 h dans 1000 ml de tampon pH 6, sous agitation 300 t/mn, à 40°C. Le taux de libération représente la proportion centésimale de principes actifs libérés au cours du test. La taille de la prise d'essai est fonction de la nature du principe actif et de sa teneur. Pour les acides aminés cités en exemples, le test est réalisé sur environ 6 g d'équivalent.

Le taux de protection doit naturellement être le plus élevé possible.

Le délitement des "pellets spéciaux selon l'invention" dans le rumen qui permet la restitution des principes actifs protégés, est évalué par incubation dans le rumen de bovins fistulés au moyen de sachets en tissu de nylon de vide de maille de 350 microns. Le temps de délitement doit être le plus faible possible, et en particulier inférieur à 48 h.

La présente invention sera plus complètement décrite à l'aide des exemples suivants qui ne peuvent être considérés comme limitatifs, tant en ce qui concerne les principes actifs protégés, que les liants utilisés.

Les principes actifs protégés utilisés pour les exemples sont des granulés d'environ 2 mm de diamètre composés de:

Principe actif protégé de type A:

Particules de lysine monochlorhydrate et de méthionine dans la proportion pondérale 75/25, contenant environ 12% d'un liant constitué par un mélange d'acide stéarique à 5 % pp de copolymère vinyl-2-pyridine co styrène, protégé contre la dégradation dans le rumen par une couche d'enrobage représentant 16 % en poids du produit protégé, composée d'un mélange de 30 % de copolymère vinyl-2-pyridine co styrène, 60 % de talc et 10 % d'acide stéarique.

Le taux de protection des granulés de type A est de 97 %.

Principe actif protégé de type B:

Particules de lysine monochlorhydrate et de méthionine dans la proportion pondérale 75/25, contenant environ 12% d'un liant constitué par un mélange d'acide stéarique à 5 % pp de copolymère vinyl-2-pyridine co styrène, protégées contre la dégradation dans le rumen par une couche d'enrobage représentant 12 % en poids du produit protégé, composée d'un mélange de 20 % de copolymère vinyl-2-pyridine co styrène et de 80 % d'acide stéarique.

Le taux de protection des granulés de type B est de 99 %.

Les produits de type A et B, utiles pour l'alimentation du ruminant sont décrits dans les brevets US 4181708 et EP 260186.

EXEMPLE 1

2 % en poids de granulés du type A sont introduits dans un mélange de farines destiné à l'alimentation du bétail, et mis sous forme d'aliment granulé dans une presse à filière rotative KAHL après traitement à la vapeur, conformément à la méthode traditionnelle de préparation des aliments granulés. Le produit obtenu se présente sous forme de cylindres extrudés de 5 mm de diamètre et d'environ 15 mm de long.

Le taux de protection résiduel des acides aminés utilisés pour l'exemple, est de 0 % dans l'aliment granulé aprés passage dans la presse.

EXEMPLE 2

2 % de granulés du type B sont introduits dans un mélange de farines destiné à l'alimentation du bétail, et mis sous forme d'aliment granulé dans une presse à filière rotative KAHL après traitement à la vapeur, conformément à la méthode traditionnelle de préparation des aliments granulés. Le produit obtenu se présente sous forme de cylindres extrudés de 5 mm de diamètre et environ 15 mm de long.

Le taux de protection résiduel des acides aminés utilisés pour l'exemple, est de 0 % dans l'aliment granulé aprés passage dans la presse.

Les exemples 1 et 2 montrent que la mise sous forme d'aliment granulé par des méthodes traditionnelles de ce type de produit conduit à une dégradation importante à totale de leur protection contre la dégradation dans le rumen, et par suite à la perte de leur efficacité pour l'alimentation du ruminant.

EXEMPLE 3

Des granulés contenant des principes actifs protégés contre la dégradation dans le rumen et miscibles en toutes proportions à l'aliment granulé traditionnel sont obtenus de la façon suivante:

Des granulés de principe actif protégé de type A sont introduits dans les cavités d'un moule. Les cavités ont une forme tronconique de hauteur 20 mm, et de diamètre aux extrêmités 8 mm et 10 mm.

Un liant fondu est injecté sous pression dans la porosité du lit particulaire ainsi réalisé dans chaque cavité.

Le produit fini est récupéré par démoulage après prise en masse du liant au refroidissement.

Le liant utilisé pour l'exemple présente la composition suivante:

| | |
|---|---|
| acide stéarique | 33,33 % |
| acide palmitique | 22,22 % |
| carbonate de calcium | 22,22 % |

résidu de fermentation                    22,22 %
(référence EUROLYSINE PL 73)

Le taux de protection et le titre en acides aminés dans le produit final sont donnés dans le tableau I.

### EXEMPLE 4

Des granulés contenant des principes actifs protégés contre la dégradation dans le rumen de type A, miscibles en toutes proportions à l'aliment granulé traditionnel sont obtenus par la technique du moulage décrite à l'exemple 3, par utilisation d'un liant fondu composé de :

acide stéarique                    30 %
acide palmitique                   20 %
mono stéarate de glycérol          50 %

Le taux de protection et le titre en acides aminés dans le produit final sont donnés dans le tableau I.

### EXEMPLE 5

Des granulés contenant des principes actifs protégés contre la dégradation dans le rumen de type A, miscibles en toutes proportions à l'aliment granulé traditionnel sont obtenus par la technique du moulage décrite à l'exemple 3, par utilisation d'un liant fondu composé de:

acide stéarique                    16,67 %
acide palmitique                   11,11 %
mono stéarate de glycérol          27,78 %
carbonate de calcium               44,44 %

Le taux de protection et le titre en acides aminés dans le produit final sont donnés dans le tableau I.

### EXEMPLE 6

Des granulés contenant des principes actifs protégés contre la dégradation dans le rumen de type A, miscibles en toutes proportions à l'aliment granulé traditionnel sont obtenus par mélange de granulés de type A avec un liant composé de:

plâtre                  22,73 %
carbonate de calcium    45,45 %
eau                     31,82 %

Le mélange est mis sous forme de particules d'environ 20 mm sur 10 mm par coulée sur un support non adhésif, et récupération après séchage à l'étuve.

Le taux de protection et le titre en acides aminés dans le produit final sont donnés dans le tableau I.

### EXEMPLE 7

Des granulés contenant des principes actifs protégés contre la dégradation dans le rumen de type A, miscibles en toutes proportions à l'aliment granulé traditionnel sont obtenus par mélange de granulés de type A avec un liant composé de :

plâtre                       25,32 %
cellulose microcristalline   18,99 %
eau                          55,70 %

Le mélange est mis sous forme de particules d'environ 20 mm sur 10 mm par coulée sur un support non adhésif, et récupérées après séchage à l'étuve.

Le taux de protection et le titre en acides aminés dans le produit final sont donnés dans le tableau I.

### EXEMPLE 8

Des granulés contenant des principes actifs protégés contre la dégradation dans le rumen de type B, miscibles en toutes proportions à l'aliment granulé traditionnel sont obtenus par mélange de granulés de type B avec un liant composé de:

plâtre                  29,41 %
carbonate de calcium    58,82 %
eau                     41,18 %

Le mélange est mis sous forme de particules d'environ 20 mm sur 10 mm par coulée sur un support non

EP 0 437 388 B1

adhésif, et récupération après séchage à l'étuve.

Le taux de protection et le titre en acides aminés dans le produit final sont donnés dans le tableau I.

EXEMPLE 9

Des granulés contenant des principes actifs protégés contre la dégradation dans le rumen de type B, miscibles en toutes proportions à l'aliment granulé traditionnel sont obtenus par mélange de granulés de type B avec un liant composé de :

| plâtre | 25,32 % |
| cellulose microcristalline | 18,99 % |
| eau | 55,57 % |

Le mélange est mis sous forme de particules d'environ 20 mm sur 10 mm par coulée sur un support non adhésif, et récupération après séchage à l'étuve.

Le taux de protection et le titre en acides aminés dans le produit final sont donnés dans le tableau I.

EXEMPLE 10

Des granulés contenant des principes actifs protégés contre la dégradation dans le rumen de type A, miscibles en toutes proportions à l'aliment granulé traditionnel sont obtenus par mélange de granulés de type A avec un liant composé de :

| mélasse de betteraves | 76,92 % |
| oxyde de calcium | 23,08 % |

Le mélange est mis sous forme de particules d'environ 20 mm sur 10 mm par coulée sur un support non adhésif, et récupération après durcissement spontané.

Le taux de protection et le titre en acides aminés dans le produit final sont donnés dans le tableau I.

EXEMPLE 11

Des granulés contenant des principes actifs protégés contre la dégradation dans le rumen de type A, miscibles en toutes proportions à l'aliment granulé traditionnel sont obtenus par mélange de granulés de type A avec un liant composé de:

| mélasse | 71,43 % |
| gélatine | 14,29 % |
| eau | 14,29 % |

Le mélange est mis sous forme de particules d'environ 20 mm sur 10 mm par coulée sur un support non adhésif, et récupération après gélification.

Le taux de protection et le titre en acides aminés dans le produit final sont donnés dans le tableau I.

EXEMPLE 12

Des granulés contenant des principes actifs protégés contre la dégradation dans le rumen de type B, miscibles en toutes proportions à l'aliment granulé traditionnel sont obtenus par mélange de granulés de type B avec un liant composé de:

| mélasse | 71,43 % |
| gélatine | 14,29 % |
| eau | 14,29 % |

Le mélange est mis sous forme de particules d'environ 20 mm sur 10 mm par coulée sur un support non adhésif, et récupération après gélification.

Le taux de protection et le titre en acides aminés dans le produit final sont donnés dans le tableau I.

Le tableau I résume les taux de protection observés pour les principes actifs protégés utilisés dans les exemples, après mise sous forme de "pellets spéciaux selon l'invention".

Il montre également que le choix judicieux de la composition du liant permet de régler la vitesse de délitement dans le rumen des dits granulés.

Il est entendu, que la méthode de mise sous forme de pellets spéciaux selon l'invention, par agglomération en présence d'un liant, décrite dans la présente invention, peut s'appliquer à une gamme extrêmement étendue de principes actifs protégés contre la dégradation dans le rumen, par adaptation des particularités physicochimiques du liant à la nature de leur protection. Devront en particulier être pris en considération le pH du liant, sa température de mise en oeuvre, et sa compatibilité ou son pouvoir solvant vis à vis de la composition pro-

7

tectrice du principe actif protégé considéré.

Caractéristiques des produits réalisés:

TABLEAU I

| N° exemple | Nature du principe actif protégé | Taux de A ou B dans le produit final aggloméré % poids | Taux de délitement du produit final dans le rumen après: | | | Taux de protection du principe actif dans le produit final % |
|---|---|---|---|---|---|---|
| | | | 6h | 24h % poids | 48h | |
| 3 | A | 52 | | 40 | 100 | 93 |
| 4 | A | 65 | | 10 | 100 | 88 |
| 5 | A | 55 | | 10 | 60 | 92 |
| 6 | A | 29 | | | 15 | 86 |
| 7 | A | 42 | 100 | | | 99 |
| 8 | B | 28 | | | 100 | 65 |
| 9 | B | 48 | 100 | | | 93 |
| 10 | A | 35 | 100 | | | 91 |
| 11 | A | 56 | 100 | | | 89 |
| 12 | B | 40 | 100 | | | 83 |

EXEMPLE 13

Des granulés contenant des principes actifs, protégés contre la dégradation dans le rumen de type B, miscibles en toutes proportions à l'aliment granulé traditionnel sont obtenus par mélange de granulés de type B avec un liant composé de:

argile                              50 %
farine de soja                      12,5 %
solution aqueuse à 4 % de CMC*      37,5 %

le mélange est mis sous forme de particules d'environ 8 mm de diamètre par extrusion à travers une filière. Les joncs ainsi obtenus sont séchés en étuve ventilée et réduits en éléments de longueur environ 20 mm.

Le taux de protection et le titre en acides aminés protégés dans le produit final sont donnés dans le tableau 2.

EXEMPLE 14

Des granulés contenant des principes actifs protégés contre la dégradation dans le rumen de type B, miscibles en toutes proportions à l'aliment granulé traditionnel sont obtenus par mélange de granulés de type B avec un liant composé de:

| | |
|---|---|
| dolomie | 59,7 % |
| farine de soja | 14,9 % |
| solution aqueuse à 4 % de CMC* | 25,4 % |

le mélange est mis sous forme de particules d'environ 8 mm de diamètre par extrusion à travers une filière. Les joncs ainsi obtenus sont séchés en étuve ventilée et réduits en éléments de longueur environ 20 mm.

Le taux de protection et le titre en acides aminés protégés dans le produit final sont donnés dans le tableau 2.

Tableau 2:

| N° exemple | Taux de B dans le produit final aggloméré<br><br>% poids | Taux de délitement dans le rumen après<br>6h      24h      48h<br><br>% poids | | Taux de protection du principe actif dans le produit final<br>% |
|---|---|---|---|---|
| 13 | 37,5 | 100 | | 99 |
| 14 | 37,5 | 100 | | 89 |

\* CMC: carboxy méthyl cellulose, sel de sodium.

**Revendications**

1. Compositions sous forme de "pellets" contenant des principes actifs granulaires protégés vis-à-vis de la dégradation dans le rumen mais dégradables par l'action conjointe de la chaleur et de la pression, utilisées pour la supplémentation alimentaire et/ ou médicamenteuse des ruminants, libérant lesdits principes actifs dans la caillette et/ou l'intestin caractérisées en ce qu'elles sont obtenues par mélange et mise en forme du principe actif protégé sous forme granulaire et d'un liant choisi parmi les liants solubilisables, reticulables ou fusibles et éventuellement d'un agent délitant et/ou d'une charge.

2. Compositions selon la revendication 1 caractérisées en ce que le principe actif est choisi parmi les vitamines, les aminoacides, les médicaments protégés vis à vis de la dégradation dans le rumen.

3. Compositions selon la revendication 2 caractérisées en ce que le principe actif protégé est choisi parmi les aminoacides, les vitamines, les médicaments protégés par un copolymére PH sensible, un produit naturel ou un produit dégradable par voie enzymatique.

4. Compositions selon la revendication 3 caractérisées en ce que le principe actif protégé est choisi parmi les aminoacides, les vitamines, les médicaments protégés par un copolymére PH sensible à base de vinyl-pyridine et de styrène en association avec une substance hydrophobe.

5. Compositions selon la revendication 3 caractérisées en ce que le principe actif protégé est choisi parmi les aminoacides, les vitamines, les médicaments protégés par un produit naturel ou un produit dégradable par voie enzymatique choisi parmi la zéïne ou le chitosan.

6. Compositions selon l'une quelconque des revendications précédentes caractérisées en ce que le principe actif protégé se présente sous forme granulaire de diamètre moyen compris entre 0,3 et 5 mm.

7. Compositions selon la revendication 1 caractérisées en ce que le liant réticulable est constitué d'un hydrocolloïde choisi parmi les alginates, les gélatines, les dérivés cellulosiques, les polysaccharides, les mélasses ou les vinasses.

8. Compositions selon la revendication 7 caractérisées en ce que l'agent de réticulation est choisi parmi les aldéhydes pour les protéines, les sels ou oxydes de métaux di ou trivalents pour les alginates, la gomme xanthane, les mélasses, les vinasses.

9. Compositions selon la revendication 1 caractérisées en ce que le liant fusible est choisi parmi les acides gras, les alcools gras, les esters du glycerol, les polyéthylèneglycols, les paraffines, les cires naturelles ou synthétiques, les graisses animales ou végétales hydrogénées.

10. Compositions selon la revendication 1 caractérisées en ce que l'agent délitant est choisi parmi les farines de grains, les tourteaux broyés, les résidus de brasserie et de fermentation, les sous-produits des céréales et du bois, les fibres cellulosiques.


## Patentansprüche

1. Zusammensetzungen in Form von "Pellets", enthaltend granulierte Wirkstoffe. die gegenüber der Zersetzung im Pansen geschützt sind, sich jedoch durch gemeinsame Einwirkung von Wärme und Druck abbauen lassen, verwendet als Nahrungs- und/oder medikamentöse Ergänzung bei Wiederkäuern, wobei die genannten Wirkstoffe im Labmagen und/oder Darm freigesetzt werden, dadurch gekennzeichnet, daß sie durch Mischen und Formgebung des geschützten Wirkstoffes in granulierter Form und eines Bindemittels, ausgewählt unter den löslichen, vernetzbaren oder schmelzbaren Bindemitteln, und gegebenenfalls eines Zerfallhilfsmittels und/oder eines Füllstoffes erhalten werden.

2. Zusammensetzung nach Anspruch 1, dadurch gekennzeichnet, daß der Wirkstoff unter den Vitaminen, den Aminosäuren und den gegenüber der Zersetzung im Pansen geschützten Medikamenten ausgewählt wird.

3. Zusammensetzung nach Anspruch 2, dadurch gekennzeichnet, daß der geschützte Wirkstoff unter den Aminosäuren, den Vitaminen und den Medikamenten ausgewählt wird, die durch ein pH-sensibles Copolymer, ein Naturprodukt oder ein auf enzymatischem Weg abbaubares Produkt geschützt sind.

4. Zusammensetzung nach Anspruch 3, dadurch gekennzeichnet, daß der geschützte Wirkstoff unter den Aminosäuren, den Vitaminen und den Medikamenten ausgewählt wird, die durch ein pH-sensibles Copolymer auf der Basis von Vinylpyridin und Styrol, in Assoziation mit einer hydrophoben Substanz, geschützt sind.

5. Zusammensetzung nach Anspruch 3, dadurch gekennzeichnet, daß der geschützte Wirkstoff unter den Aminosäuren. den Vitaminen und den Medikamenten ausgewählt wird, die durch ein Naturprodukt oder ein auf enzymatischem Weg abbaubares Produkt geschützt sind. ausgewählt unter Zein oder Chitosan.

6. Zusammensetzung nach irgendeinem der vorstehenden Ansprüche, dadurch gekennzeichnet, daß der geschützte Wirkstoff in Form von Granulaten mit einem mittleren Durchmesser zwischen 0,3 und 5 mm vorliegt.

7. Zusammensetzung nach Anspruch 1. dadurch gekennzeichnet. daß das vernetzbare Bindemittel aus einem Hydro-Kolloid besteht, ausgewählt unter den Alginaten, den Gelatinen, den Cellulose-Derivaten, den Polysacchariden. den Melassen oder den Schlempen.

8.  Zusammensetzung nach Anspruch 7, dadurch gekennzeichnet, daß das Vernetzungsmittel unter den Aldehyden für die Proteine, den Salzen oder Oxiden von zwei- oder dreiwertigen Metallen für die Alginate, Xanthangummi, die Melassen und die Schlempen ausgewählt wird.

9.  Zusammensetzung nach Anspruch 1, dadurch gekennzeichnet, daß das schmelzbare Bindemittel unter den Fettsäuren, den Fettalkoholen, den Glycerinestern, den Polyethylenglykolen, den Paraffinen, den natürlichen oder synthetischen Wachsen oder den hydrierten. tierischen oder pflanzlichen Fetten ausgewählt wird.

10. Zusammensetzung nach Anspruch 1, dadurch gekennzeichnet, daß das Zerfallhilfsmittel unter den Getreidemehlen, den zerkleinerten Ölkuchen, den Rückständen von Brauerei und Fermentation, den Nebenprodukten von Getreide und Holz oder den Cellulosefasern ausgewählt wird.

## Claims

1.  Compositions in the form of "pellets" containing granular active principles protected against degradation in the rumen but which are degradable through the joint action of heat and pressure, used for the nutritional and/or medicinal supplementation of ruminants, releasing the said active principles in the abomasum and/or the intestine, characterized in that they are obtained by mixing and shaping the protected active principle in granular form and a binding agent selected from binding agents capable of being solubilized, crosslinked or melted, and optionally a disintegrating agent and/or a filler.

2.  Compositions according to claim 1, characterized in that the active principle is selected from vitamins, amino acids and drugs protected from degradation in the rumen.

3.  Compositions according to claim 2, characterized in that the protected active principle is selected from amino acids, vitamins and drugs protected by a pH-sensitive copolymer, a natural product or an enzymatically degradable product.

4.  Compositions according to claim 3, characterized in that the protected active principle is selected from amino acids, vitamins and drugs protected by a pH-sensitive copolymer based on vinylpyridine and on styrene in combination with a hydrophobic substance.

5.  Compositions according to claim 3, characterized in that the protected active principle is selected from amino acids, vitamins and drugs protected by a natural product or an enzymatically degradable product selected from zein or chitosan.

6.  Compositions according to any one of the preceding claims, characterized in that the protected active principle takes a granular form of average diameter between 0.3 and 5 mm.

7.  Compositions according to claim 1, characterized in that the binding agent capable of being crosslinked consists of a hydrocolloid selected from alginates, gelatins, cellulose derivatives, polysaccharides, molasses or vinasses.

8.  Compositions according to claim 7, characterized in that the crosslinking agent is selected from aldehydes for proteins, salts or oxides of di- or trivalent metals for alginates, xanthan gum, molasses and vinasses.

9.  Compositions according to claim 1, characterized in that the binding agent capable of being melted is selected from fatty acids, fatty alcohols, glycerol esters, polyethylene glycols, paraffin waxes, natural or synthetic waxes and hydrogenated animal or vegetable fats.

10. Compositions according to claim 1, characterized in that the disintegrating agent is selected from grain flours, ground feed cakes, brewery and fermentation residues, cereal and wood by-products and cellulose fibres.